# EUROPEAN PATENT APPLICATION

(11) **EP 4 616 791 A1**
(43) Date of publication of application: **17.09.2025**
(21) Application number: 24205660.4
(22) Date of filing: 09.10.2024
(51) Int. Cl.: A61B 5/00, A61F 5/56

(54) **A METHOD FOR DESIGNING AND MANUFACTURING A CUSTOM SENSORIZED ORAL APPLIANCE FOR MANAGING SLEEP APNEA, AND A CUSTOM SENSORIZED ORAL APPLIANCE FOR MANAGING SLEEP APNEA**

(30) Priority: 15.03.2024 EP 24163853
(71) Applicant: Aesyra SA, 1015 Lausanne (CH)
(72) Inventor: Letizia, Marco, 1024 Ecublens (CH); Maoddi, Pietro, 1025 Saint Sulpice (CH); Altruda, Samuele, 1024 Ecublens (CH)
(74) Representative: P&TS SA (AG, Ltd.)

(57) **Abstract**

The present invention concerns a custom sensorized oral appliance (8) for managing sleep apnea, comprising:
- a main body of the oral appliance, the main body comprising:
- a custom cavity (9) arranged to host a custom electronic element (20),
- the custom electronic element comprising:
- a rigid part (1), and
- a sleep apnea sensor (3), and.

The present invention concerns also a method for designing and manufacturing the oral appliance (8), the oral appliance (8) and a kit for managing sleep apnea.

## Description

### Technical domain

The present invention concerns a method for designing and manufacturing a custom sensorized oral appliance for managing sleep apnea, and a custom sensorized oral appliance for managing sleep apnea.

### Related art

Sleep apnea is a serious sleep disorder where a person's breathing repeatedly stops and starts during sleep. The two main types are obstructive sleep apnea (OSA), which occurs when throat muscles intermittently relax and block the airway, and central sleep apnea (CSA), where the brain fails to send proper signals to muscles controlling breathing. The most common is OSA, which can lead to fragmented sleep, resulting in excessive daytime sleepiness, fatigue, and difficulty concentrating. Untreated, it is linked to more severe health issues like cardiovascular disease, stroke, and hypertension.

Treatment options vary, but the most common is the use of Continuous Positive Airway Pressure (CPAP) machines, which keep airways open by delivering constant air pressure through a mask. However, compliance with CPAP devices is often low due to discomfort, noise, or inconvenience.

Other treatments include oral appliances that adjust the position of the jaw and/or tongue. Sensorized oral appliance for managing sleep apnea are known. However, they have some limitations, including user discomfort and/or bulkiness, complex clinical workflow to adjust the jaw position, and limiting capability in monitoring continuously the patient sleep data.

Custom designs of oral appliances are also known. In dentistry, the term "custom" typically refers to something that is made specifically for a user patient based on their unique dental anatomy and/or needs. This could include custom-made dental appliances such as crowns, bridges, dentures, orthodontic appliances, mouthguards, or oral appliances. To create custom dental appliances, dental professionals often take detailed impressions or 3D digital scans of the user's teeth and gums. These impressions or scans are then used to fabricate the oral appliance.

In this context the oral anatomy of a user comprises at least a portion of its oral cavity, including the maxillary dental arche and/or mandibular dental arche of the user. It might include also parameters extracted from the anatomy, as the curvature of dental arches, angles of teeth, distances between cusps, etc.

Moreover, the manufacturing process is complex and cannot be as automated as possible.

### Short disclosure of the invention

An aim of the present invention is the provision of a method for designing and manufacturing a custom sensorized oral appliance for managing sleep apnea, and a custom sensorized oral appliance for managing sleep apnea, that overcome the shortcomings and limitations of the state of the art.

Another aim of the invention is the provision of a method for designing and manufacturing a custom sensorized oral appliance for managing sleep apnea, which allows to maximize performances in sleep apnea detection.

Another aim of the invention is the provision of a method for designing and manufacturing a custom sensorized oral appliance for managing sleep apnea, wherein the oral appliance is more comfortable than known solutions.

Another aim of the invention is the provision of a method for designing and manufacturing a custom sensorized oral appliance for managing sleep apnea, wherein the oral appliance is as small as possible. Aspects of the management are also related to jaw advancement and continuous monitoring of the patient's sleep data

Another aim of the invention is the provision of a method for designing and manufacturing a custom sensorized oral appliance for managing sleep apnea, alternative to the state-of-the-art solutions.

An optional aim of the invention is the provision of a method for designing and manufacturing a custom sensorized oral appliance for managing sleep apnea, simplifying the manufacturing process.

An optional aim of the invention is the provision of a method for designing and manufacturing a custom sensorized oral appliance for managing sleep apnea, wherein the manufacturing process is as automated as possible.

An optional aim of the invention is the provision of a method for designing and manufacturing a custom sensorized oral appliance for managing sleep apnea, wherein the sealing is better than known solutions.

An optional aim of the invention is the provision of a method for designing and manufacturing a custom sensorized oral appliance for managing sleep apnea, wherein the oral appliance is more reliable and safer for the user.

According to the invention, these aims are attained by the object of the attached claims, and especially by a method for designing a custom sensorized oral appliance for managing sleep apnea of a user according to claim 1, by a method for manufacturing a custom sensorized oral appliance for managing sleep apnea of a user according to claim 4, by a custom sensorized oral appliance for managing sleep apnea of a user according to claim 8 and by a kit for managing sleep apnea according to claim 12, wherein preferred embodiments of the invention are given in the dependent claims.

The method for designing a custom sensorized oral appliance for managing sleep apnea of a user according to the invention, comprises the steps of:
- collect data on the 3D oral anatomy of the user, e.g. from a 3D anatomical scan, from a 3D representation of a custom oral appliance, etc.
- computationally design a main body of the oral appliance, the main body comprising:
   - a cavity arranged to host an electronic element, the cavity being customized on the basis of the data and/or based on a computationally designed electronic element,
   - computationally design the electronic element, the electronic element comprising:
   - a rigid part, and
   - a sleep apnea sensor for detecting a sleep apnea event,
   wherein the spatial placement of the rigid part, and/or of the sleep apnea sensor is based on the data.

In this context, a sleep apnea sensor is a sensor adapted for detecting a sleep apnea event. The sleep apnea sensor can be for example:
- A pressure sensor, arrange for airflow measurement: it measures the pressure changes in the user's airway or mouth during breathing, and can detect obstructed airflow by monitoring changes in pressure, helping to identify apnea events.
- An oxygen saturation sensors (or pulse oximeter): it measures the oxygen levels in the blood, providing real-time data on oxygen saturation (SpO2). It can be used to detect drops in oxygen levels during apnea episodes, helping monitor the severity of the condition. It can be an optical or reflective sensors that can be embedded into the appliance.
- A thermocouples or temperature sensor, for airflow detection): it measures temperature variations caused by exhaled air. It can monitor the airflow and detect apnea by sensing the absence or reduction of warm, exhaled air from the mouth or nose. It can be a small thermocouple sensor arranged to detect temperature changes as the user breathes.
- An accelerometer (for detecting body and/or jaw movements): it detects movement or position changes in the jaw, mouth, or head. It can monitor jaw position and detect changes during apnea events, particularly useful in detecting obstructive apnea caused by jaw misalignment. It can be e.g. an embedded accelerometer to track jaw movements or orientation during sleep.
- An EEG/EOG sensor, for sleep stage monitoring): it measures electrical activity of the brain (EEG) or eye movements (EOG). It can be used to monitor sleep stages and correlate the occurrence of apnea events with specific sleep phases (e.g., REM sleep). It can be an ultra-thin electrodes embedded in the dental appliance.
- A microphone or sound sensor, for snoring detection: it detects sound, particularly snoring, which often accompanies obstructive sleep apnea. It can identify patterns of snoring that indicate apnea events. It can be a small, embedded microphones in the dental appliance that capture the sound of snoring and pauses in breathing.
- A biosensor, for saliva and/or pH monitoring: it measures biochemical markers in saliva, such as pH or hydration levels. It tracks physiological changes related to apnea episodes, like stress responses or dehydration, which might be associated with sleep disturbances. It can be a saliva sensor embedded in the dental appliance to monitor biochemical fluctuations during sleep.
- A capnometers for CO2 measurement: it measures exhaled carbon dioxide (CO2) levels. It tracks CO2 concentration in breath to detect inadequate ventilation during sleep, a sign of apnea. It can be a miniature CO2 sensor to monitor exhaled gases.

The detection can be performed by analyzing one or multiple parameters in time.

In this context, the sleep apnea sensor is not a force sensor, in particular is not a force sensor or the transduction of occlusal forces produced between teeth during bruxism.

The apnea sensor, when embedded in a dental appliance, could provide real-time monitoring, diagnostics, and data collection, improving the appliance's capability to track and respond to sleep apnea events.

In one embodiment, the custom sensorized oral appliance according to the invention comprises two or more apnea sensors, which can be equal or different.

In one embodiment the design comprises the modification of the main body of the oral appliance.

In this context, the design is performed "computationally", i.e. with a computing module.

In one embodiment, the computing module is a machine learning-based module, including, but not limited to algorithm of 3D Pose Estimation, Object Classification, Objects Detection, Scene/Object Semantic Segmentation, 3D Geometry Synthesis/Reconstruction, Deep Learning Methods, Texture/Material Analysis, or any combination of thereof.

In this context, a machine learning-based module is a module which needs to be trained in order to learn i.e. to progressively improve a performance on a specific task. In one embodiment, the machine learning-based module is an artificial neural network, or neural network for short. It comprises a set of artificial neurons that are connected to each other to form a weighted directed graph. The neurons receive inputs from other neurons that feed into them, operate on these inputs, and transmit the results to the neurons they feed into. The edge weights denote the influence that a neuron has on those it is connected to and are computed by a process called learning which involves minimizing a loss function.

The computationally designed custom sensorized oral appliance according to the invention, including the computationally designed electronic element, comprising a rigid part, a sleep apnea sensor and a flexible electronic interconnection, and including a computationally generated cavity hosting the electronic element and the bending of its flexible electronic interconnection, allows to maximize performances in sleep apnea detection.

The cavity of the main body of the oral appliance allows the components of the electronic elements to remain in the "right" place and to be operatively connected, in particular during sleep apnea events.

In one embodiment, the electronic element comprises a flexible electronic interconnection, arranged for electronically connect the rigid part with a distal element in the appliance or with a distal device in the appliance.

In one embodiment, the flexible electronic interconnection is arranged for electronically connect the rigid part with the sleep apnea sensor.

Since the spatial placement in the electronic element of the rigid part, and/or of the sleep apnea sensor and/or the bending of the flexible electronic interconnection are based on the data on the 3D oral anatomy of the user, the space for those components is minimised, thereby rendering the oral appliance as small as possible.

In one preferred embodiment, the rigid part of the electronic element comprises components preferably not to be bent for reliability reasons, such as integrated circuits, microcontrollers, discrete electronic components, batteries, digital memories, radio-frequency antennas, coils for receiving wireless power, contacts for receiving power and/or exchanging data, sensors such as accelerometers, actuators to produce vibrations and/or sound, etc. In this context, a flexible electronic interconnection is a bendable electronic interconnection, arranged to create an electronic connection for exchanging power and/or data.

In one embodiment, the rigid part comprises the sleep apnea sensor.

In one embodiment, the electronic element comprises a feedback module, arranged to send feedback to the user so as to create real-time awareness of breathing irregularities that cause snoring, and over time, help the user make subconscious adjustments to their breathing patterns, wherein the activation of the feedback module is in particular based on the sleep apnea sensor data.

In one embodiment, the spatial placement of the feedback module in the appliance is based on the data on the 3D oral anatomy of the user.

In one embodiment, the feedback module is based on or comprises an audio / vibration sensor with haptic feedback: this sensor generates a (subtle) haptic feedback (e.g., gentle vibration, audio sound, or bone conduction stimulus) through the dental appliance.

In one embodiment the feedback module comprises a microelectromechanical systems (MEMS) sensor. In one embodiment the feedback module is arranged to send feedback signals through integrated speakers or vibration pads. In one embodiment, the feedback mechanism is based on an electrical stimulation.

In one embodiment, the rigid part comprises a microprocessor arranged to receive the sleep apnea sensor data, to process them and, based on this processing, to send an activation signal to the feedback module, so as to activate it. This embodiment is preferred, since it is simpler for regulatory reasons, and it does not depend on the kind of external processor that could be used instead.

In another embodiment, sleep apnea sensor data are sent to an external processor, arranged to process them and, based on this processing, to send an activation signal to the feedback module, so as to activate it.

In one embodiment, the cavity comprises special structures, voids, etc.

In one embodiment, the main body comprises an insertion port, arranged for inserting the electronic element into the main body.

In this context, the insertion port is an interface used to insert the electronic element inside the oral appliance, which preferably needs to be sealed to prevent the ingress of liquids (e.g. saliva) during use and to prevent tampering with the electronic element.

In one embodiment, the insertion port has a shape and/or dimensions which are standard, i.e. independent on the oral anatomy of the user. For example, the insertion port could be a standard through opening of the main body. Its spatial position could be dependent on the oral anatomy of the user. In this case, the insertion port could be at least a part of the region of the cavity arranged to host the rigid part of the electronic element.

In another embodiment, the insertion port has a shape, dimensions and a spatial placement which depend on the oral anatomy of the user. For example, the insertion port is a volume completely included in the main body. In one embodiment, during the manufacturing the electronic element is inserted into the cavity formed between a liner and a frame, the inlet of which is an insertion port dependent on the oral anatomy of the user. In another embodiment, an additive manufacturing device as a 3D printer creates the frame, the additive manufacturing is paused to insert the electronic element, and finally the additive manufacturing is completed by sealing the oral appliance. In this case, the shape of the insertion port is not standard. In this case, the insertion port could comprise a part of the region of the cavity arranged to host the sleep apnea sensor and a part of the region of the cavity hosting flexible electronic interconnections, and a part of the region of the cavity arranged to host the rigid part of the electronic element.

If an insertion port having standard shape and/or standard dimensions is present, i.e. having a shape and/or dimensions which are independent on the oral anatomy of the user (this insertion port being referred to in the following as a "standard insertion port"), the sensorized oral appliance is customized but at the same time it comprises a standard insertion port which shape and/or dimensions do not depend on the oral anatomy of the user. This allows standard electronic elements to be inserted in the insertion port of any custom oral appliance. This simplifies and renders as automated as possible the manufacturing of the oral appliance. Moreover, since the insertion port is standard, standard lids can be used to seal any custom sensorized oral appliance oral appliance. Finally, previously designed and tested methods of securely attaching and sealing the lid on the oral appliance can be used as well.

The standard geometry of the insertion port has also the advantage of decreasing the possibility of introducing defects in the seal during the sealing process and allowing a more reliable, possibly automated, inspection of the seal. These features ultimately lead to an oral appliance that is more reliable and safer for the user. Moreover, the position of the sealing components (port) can be optimized to avoid structural deformation during sleep apnea or during use to avoid failure and liquid penetration.

In one embodiment, the method for designing a custom sensorized oral appliance for managing sleep apnea of a user the comprises the step of choosing a length of the flexible electronic interconnection from a library of standard values, corresponding to available sizes of the electronic element, or computing a customized length of the flexible electronic interconnection.

In one embodiment, the method for designing a custom sensorized oral appliance for managing sleep apnea of a user comprises the step of designing the region of the cavity hosting the sleep apnea sensor(s) between a liner, arranged to be in contact with the teeth of the arch on which the oral appliance is worn, and a frame arranged to be in contact with the teeth of the antagonist dental arch.

In this context, a linear is a part of the oral appliance in contact with the dental arch on which the oral appliance is worn. The liner is typically made of a softer material to provide higher deformation of the cavity and/or a better grip on the teeth.

In this context, a frame is a part of the oral appliance that faces the dental arch antagonist to the one on which the appliance is worn. The frame is typically made of a harder and stiffer material in order to resist mechanical wear and avoid a "chewy" sensation to the user of the appliance.

In one embodiment, the electronic element is sandwiched between the liner and the frame.

In one embodiment, the sealing of the oral appliance is obtained by bonding the liner and the frame together. In this embodiment, a lid can be present or not.

In one embodiment, the method for designing a custom sensorized oral appliance for managing sleep apnea of a user the comprises the step of designing the region of the cavity hosting the sleep apnea sensor, so that it is entirely contained within the liner or the frame.

In one embodiment, the rigid part is a first rigid part, and the electronic element comprises a second rigid part and a second flexible electronic interconnection arranged for electronically connect the first rigid part with the second rigid part.

The method for manufacturing a custom and sensorized oral appliance for managing sleep apnea of a user according to the invention comprises the steps of:
- designing the oral appliance with the method for designing a custom sensorized oral appliance for managing sleep apnea of a user according to the invention,
- manufacturing the main body of the oral appliance and the electronic element.

In one embodiment, the method for manufacturing a custom and sensorized oral appliance comprises the step of inserting the electronic element into the main body via the insertion port.

In one embodiment, the method for manufacturing a custom and sensorized oral appliance comprises the step of sliding the electronic element into its rest operating position in the cavity.

In one embodiment, the insertion port comprises a hole and the flexible electronic interconnection is inserted in its receiving cavity region through this hole.

In one embodiment, the method for manufacturing a custom and sensorized oral appliance comprises the step of the deformation (e.g. by elongation) of the flexible electronic interconnection of the electronic element during its insertion and/or sliding.

In one embodiment, the sleep apnea sensor and the rigid part are inserted in their respective receiving cavity regions through the insertion port.

In one embodiment, the manufacturing is performed with additive processes, for example 3D printing, selective laser sintering, stereolithography, etc.

In one embodiment, the manufacturing is performed with subtractive processes, for example CNC machining, electro-discharge machining, etc.

In one embodiment, the method for manufacturing a custom and sensorized oral appliance for managing sleep apnea of a user comprises the step of changing the stiffness of the cavity using the same material, therefore changing the response of the sleep apnea sensor, e.g. to the applied force.

In one embodiment, the method for manufacturing a custom and sensorized oral appliance for managing sleep apnea of a user comprises the step of closing the insertion port with a lid.

In one embodiment, the method for manufacturing a custom and sensorized oral appliance for managing sleep apnea of a user comprises the step of sealing the oral appliance once the lid has been attached and/or the step of sealing the liner and the frame together, e.g. by vapor smoothing, chemical smoothing, abrasive methods, heat polishing, filling materials, mechanical polishing, chemical vapour deposition processes, etc.

In one embodiment, the insertion port is a first port and the method for manufacturing a custom and sensorized oral appliance for managing sleep apnea of a user comprises the step of recovering an end of the flexible electronic interconnection from a second insertion port opposite to the first insertion port.

In one embodiment, the method for manufacturing a custom and sensorized oral appliance for managing sleep apnea of a user comprises the step of connecting the end of the flexible electronic interconnection to a second rigid part of the electronic element, e.g. via a connector, thus completing the electronic connection. The assembly of the oral appliance can be completed by inserting a second sleep apnea sensor and a second rigid part in their respective receiving cavity regions through the second insertion port, which can be finally sealed closed, e.g. by the second lid.

In one embodiment, the sealing between the lid and the oral appliance can be obtained using an O-ring and/or mechanical snap-in features on the lid.

In one embodiment, the sealing can be obtained by attaching the lid to the oral appliance, e.g. through the use of an adhesive, for example cyanoacrylate glue, epoxy glue, hybrid glue, hotmelt, etc.

In one embodiment, the lid is a pre-integrated lid, for example by injection over-molding of plastic, with the electronic element.

In one embodiment, the lid is overmolded on a rigid part of the electronic element, in such a way that electronic contacts for exchanging power and/or data are integrated in the lid.

In one embodiment, the entire outer surface of the lid is metallic and is used as an electronic contact for exchanging power and/or data.

In one embodiment, power and/or data can be wirelessly transferred to the oral appliance.

In one embodiment, the cavity is obtained between two materials of different stiffness.

In one embodiment, the cavity is obtained within a homogenous material.

In one embodiment, part or the entirety of the electronic element is partially or entirely encapsulated in a sealing material, e.g. parylene-coated, injection over-molded with plastic, etc.

In one embodiment, the oral appliance is made of one plastic material or multiple plastic materials.

The custom sensorized oral appliance for managing sleep apnea of a user according to the invention comprises:
- a main body of the oral appliance, the main body comprising:
   - a customised cavity arranged to host a customised electronic element,
   - the customised electronic element comprising:
   - a rigid part,
   - a sleep apnea sensor.

In one embodiment, the custom sensorized oral appliance comprises a flexible electronic interconnection, arranged for electronically connect the rigid part with a distal element in the appliance or a distal device in the appliance.

In one embodiment, the flexible electronic interconnection is arranged for electronically connect the rigid part with the sleep apnea sensor..

In this context, an electronic element is custom or customized, if the spatial placement in the electronic element of the rigid part, and/or of the sleep apnea sensor and/or the bending of the flexible electronic interconnection is(are) based on the data on the 3D oral anatomy of the user.

In one embodiment, the custom sensorized oral appliance is a first custom sensorized oral appliance, comprising a communication module arranged to send an information based on the sleep apnea sensor data to a second custom sensorized oral appliance or to an external device.

In one embodiment, the first custom sensorized oral appliance comprises a coupling module, arranged to identify which second appliance the user is wearing from those available and/or its advancement with regard to the first custom sensorized oral appliance.

The present invention concerns also a kit for managing sleep apnea comprising the first custom sensorized oral appliance according to the invention and at least a second oral appliance.

In one embodiment the second oral appliance is custom oral appliance according to the invention, with the difference that it can be devoid of a sleep apnea sensor.

In one embodiment, the second oral appliance comprises a communication module arranged to receive the information based on the sleep apnea sensor data from the first custom sensorized oral appliance.

In one embodiment, the second oral appliance comprises an actuating module arranged to move the second oral appliance relatively to the first custom sensorized oral appliance, based on the received information based on the sleep apnea sensor data from the first custom sensorized oral appliance.

In one embodiment, the first custom sensorized oral appliance and/or the second oral appliance comprise(s) a coupling module, arranged to identify by the first custom sensorized oral appliance, which second appliance the user is wearing from those available in the kit.

In one embodiment, the kit comprises at least two (e.g. three) second custom sensorized oral appliances with different lengths or advancements (titration), allowing different adjusting of the position of the jaw and/or tongue of the user.

In one embodiment, an arm is arranged to connect the first custom sensorized oral appliance with the second oral appliance and/or to relatively move the second oral appliance with regard to the first custom sensorized oral appliance.

### Short description of the drawings

Exemplar embodiments of the invention are disclosed in the description and illustrated by the drawings in which:
Figure 1 illustrates schematically a front view of the electronic element according to one embodiment of the invention.
Figure 2 illustrates schematically a perspective view of a model of the electronic element and of a part of the 3D oral anatomy of the user according to one embodiment of the invention.
Figure 3A illustrates schematically a perspective view of the oral appliance worn by a user, according to one embodiment of the invention.
Figure 3B illustrates schematically the external aspect of the oral appliance of Figure 3A, worn by a user.
Figure 4A illustrates schematically a perspective view of the oral appliance worn by a user, according to one embodiment of the invention.
Figure 4B illustrates schematically the external aspect of the oral appliance of Figure 4A, worn by a user.

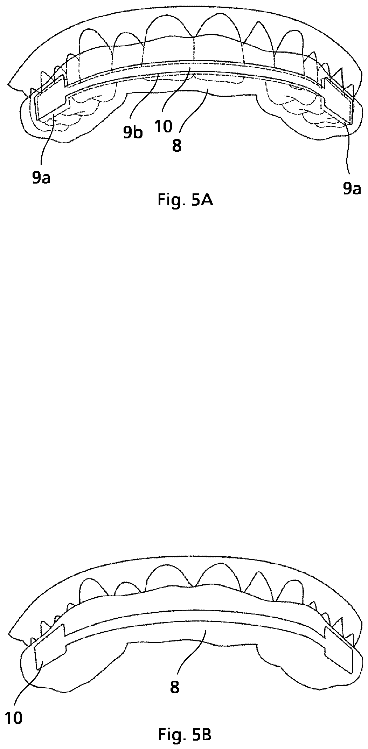

Figure 5A illustrates schematically a perspective view of the oral appliance worn by a user, according to one embodiment of the invention.
Figure 5B illustrates schematically the external aspect of the oral appliance of Figure 5A, worn by a user.
Figure 6 illustrates schematically a partially exploded perspective view of the oral appliance according to one embodiment of the invention.
Figure 7 illustrates the oral appliance of Figure 6 with another perspective view.
Figure 8 illustrates schematically a partially exploded perspective view of the oral appliance according to one embodiment of the invention.
Figure 9 illustrates schematically a cross-section view of a portion of the oral appliance according to one embodiment of the invention.
Figure 10 illustrates schematically a cross-section view of a portion of the oral appliance according to one embodiment of the invention.
Figure 11 illustrates schematically a perspective view of a portion of the oral appliance according to one embodiment of the invention.
Figure 12 illustrates schematically a perspective view of a first oral appliance according to one embodiment of the invention and of a second oral appliance.
Figure 13 illustrates schematically a perspective view of a first oral appliance according to another embodiment of the invention and of a second oral appliance, this embodiment being dual to the one of Figure 12.

### Examples of embodiments of the present invention

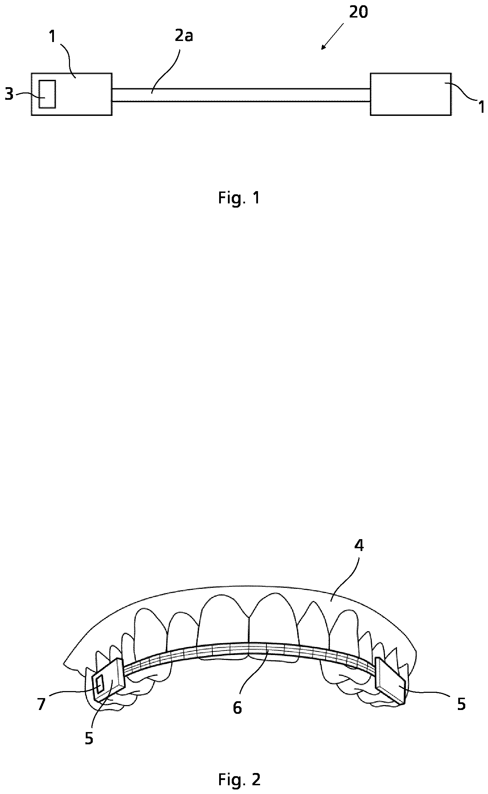

The oral appliance 8 according to the invention comprises an electronic element 20, an embodiment of which is illustrated in Figure 1.

The electronic element 20 comprises at least one rigid part 1, two in the embodiment of Figure 1. Each rigid part 1 is not-bendable and typically containing components preferably not to be bent for reliability reasons, such as integrated circuits, microcontrollers, discrete electronic components, batteries, digital memories, radio-frequency antennas, coils for receiving wireless power, contacts for receiving power and/or exchanging data, sensors such as accelerometers, actuators to produce vibrations and/or sound, etc.

The electronic element 20 comprises flexible electronic interconnection 2a between the rigid parts 1. The flexible electronic interconnection 2a cannot be present in embodiments where only one rigid part 1 is included in the electronic module 20. The flexible electronic interconnection 2a is bendable and can include some components.

The electronic element 20 also includes at least one sleep apnea sensor 3, two in the embodiment of Figure 1, connected to the rigid parts 1 via a flexible electronic interconnection 2b. Also, the flexible electronic interconnection 2b is bendable and can include some components.

In one embodiment, if the electronic element 20 is not yet mounted in the oral appliance, the flexible electronic interconnection 2a is substantially perpendicular to the flexible electronic interconnections 2b, the rigid part 1 is substantially coplanar with the sensor 3, and the flexible electronic interconnections 2a, 2b are linear.

Part or the entirety of the electronic element 20 can be partially or entirely encapsulated in a sealing material, e.g. parylene-coated, injection over-molded with plastic.

In one embodiment, the spatial placement of the rigid parts 1 and of the sleep apnea sensors 3, and the bending of the flexible electronic interconnections 2a, 2b, is computationally designed using models of the electronic element 5, 7 and of the oral anatomy of the user 4, in particular including a model of the 3D oral anatomy of the user, e.g. collected by an anatomical scan, including 3D models of the maxillary and mandibular dental arches. In Figure 2, only the maxillary arch is shown for clarity.

In one embodiment, once the electronic element 20 is mounted in the oral appliance, the rigid part 1 forms an angle with the sensor 3. This angle is obtained by bending the flexible electronic interconnections 2b.

In another embodiment, once the electronic element 20 is mounted in the oral appliance, the rigid part 1 is coplanar with the sensor 3.

In one embodiment, once the electronic element 20 is mounted in the oral appliance, flexible electronic interconnection 2a is curved. This is obtained by bending the flexible electronic interconnection 2a.

In some embodiments, an optimal length of flexible electronic interconnection models can be chosen from a library of standard values, corresponding to available sizes of the electronic element 20, or calculated a custom length for such interconnections, corresponding to the length at which a fixed-size electronic substrate needs to be cut to adapt to the electronic modules' distance in the oral appliance 8.

The main body of the oral appliance 8 is also computationally designed, by featuring a cavity 9 for the placement of the electronic element 20. In one embodiment, the cavity 9 is designed based on the design of the electronic element 20.

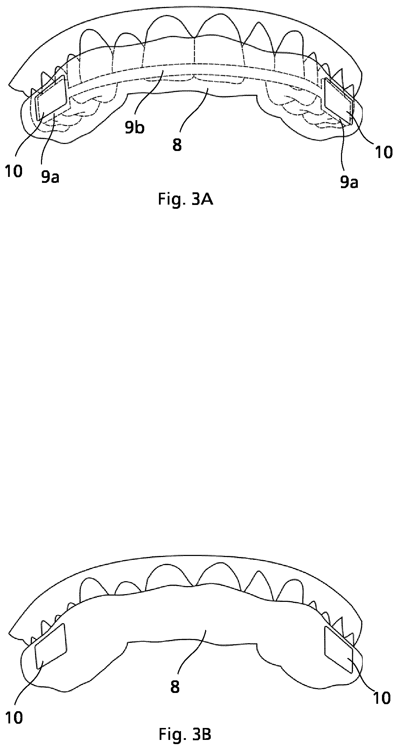

In one embodiment, visible e.g. in Figure 3A, the cavity 9 comprises a region 9a for the placement of the rigid parts, a region 9b for the placement of the flexible electronic interconnections and possibly a region for the placement of the sleep apnea sensor.

The cavity 9 allows the components of the electronic module 20 to remain in the right place and operatively connected, in particular during sleep apnea events.

The cavity 9 allows also the electronic element 20, comprising the sensor 3, to slide during the mounting phase into the rest operating position.

The cavity 9 allows also the electronic element 20 to be host in place during the rest operating position and comply with possible deformation during the sensor compression.

The cavity 9 allows also the electronic element 20 to deform to comply with elongation needed during the mounting phase.

In one embodiment, the main body of the oral appliance 8 includes at least one insertion port, which in one embodiment is an opening to be used for the assembly of the electronic element 20 into the oral appliance 8.

In one embodiment, the insertion port is closed by a lid 10, e.g. during the final assembly of the oral appliance 8.

In one embodiment, the lid 10 seals at least one end of the cavity 9.

In one embodiment, the lid 10 holds a (press-fit) O-ring to seal the closure.

In one embodiment, the lid 10 a gap with the oral appliance that is filled with a sealing agent.

In one embodiment, the oral appliance 8 is manufactured with an additive process, for example 3D printing, selective laser sintering, stereolithography, etc.

In one embodiment, the oral appliance 8 is manufactured with a subtractive process, for example CNC machining, electro-discharge machining, etc.

In one embodiment, the oral appliance 8 features cavities or a housing for the sleep apnea sensor. In one embodiment, the sleep apnea sensor is in or on the rigid part 1.

In one embodiment, the oral appliance 8 comprises geometrical structures to change the stiffness of the cavities using the same material, therefore changing the response of the sleep apnea sensors to the applied force.

In one embodiment, the cavity 9 is obtained between two materials of different stiffness rather than within a homogenous material.

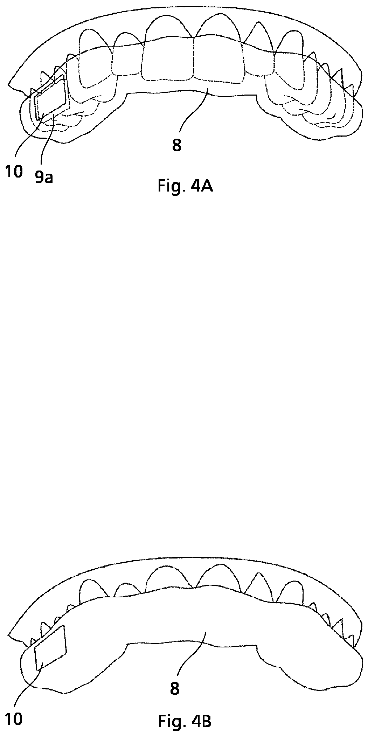

In one embodiment, the cavity region 9a containing the flexible electronic interconnections between rigid parts is completely buried in the material, as illustrated in Figure 4.

In one embodiment, the cavity region 9a containing the flexible electronic interconnections between rigid parts is absent in reason of there being only one rigid part.

In one embodiment, the cavity region 9a containing the flexible electronic interconnections between rigid parts is shaped as to present an opening in correspondence of the appliance surface, effectively becoming part of the insertion ports for the electronic element 20, which is sealed closed by lids 10 during the final assembly of the device.

The manufacturing of the oral appliance 8, and in particular its assembly, can be performed in several ways.

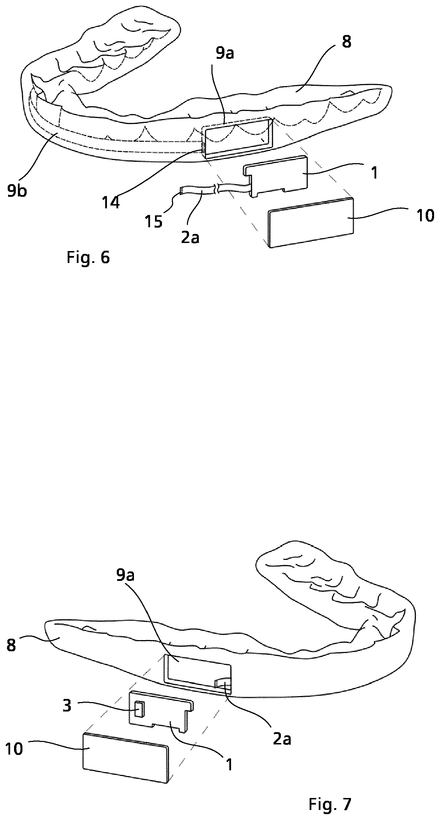

In one embodiment, illustrated in Figure 6, the flexible electronic interconnection 2a stemming from a first rigid part 1 is initially open-ended, terminating in a connector 15. Such flexible electronic interconnection 2a is inserted in its receiving cavity region 9b of the cavity 9 through a hole 14 connecting regions 9a and 9b of the cavity 9, while a first sleep apnea sensor 3 and the first rigid part 1 are inserted (e.g. in their respective receiving cavity regions) through a first insertion port represented by the open face of cavity 9a. Such insertion port can be sealed closed by a lid 10.

In one embodiment, illustrated in Figure 7, the assembly continues by recovering the open-ended flexible electronic interconnection 2a from the opposite side of the oral appliance 8. Such flexible electronic interconnection 2a can be then connected to a second rigid part 1 of the electronic element via the connector 15, thus completing the electronic connection.

The assembly of the oral appliance 8 can completed by inserting the second sleep apnea sensor 3 and the second rigid part 1 (e.g. in their respective receiving cavity regions) through the second insertion port represented by the open face of cavity 9a, which can be finally sealed closed by the second lid 10.

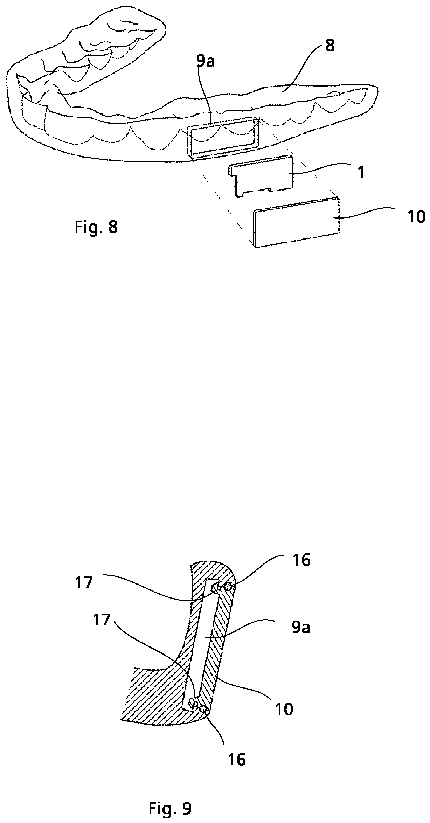

In another embodiment, illustrated in Figure 8, the oral appliance is devoid of the flexible electronic interconnection 2a and the single sleep apnea sensor 3 and the rigid part 1 are inserted (e.g. in their respective receiving cavity regions) through the insertion port represented by the open face of cavity 9a, which can be finally sealed closed by the second lid 10.

In one embodiment the oral appliance 8 comprises insertion port(s) and possible lid(s), the shape and/or dimensions of which are standard, i.e. they do not depend on the oral anatomy of the user. This present several advantages:
- standard electronic element(s) 20 can be inserted in the insertion ports of any custom oral appliance 8,
- standard lid(s) 10 can be used to seal any custom oral appliance 8,
- previously designed and tested methods of securely attaching and sealing the lid 10 on the oral appliance 8, can be used such as screws, press-fit or snap-in mechanical joints or (automatically applied) adhesives, possibly in combination with gaskets or O-rings to improve water-proofness of the sealing. Any combination of the previously designed and tested methods of securely attaching and sealing the lid 10 on the oral appliance 8 can be used as well.

The small size (the size ranging from hundreds of micrometres to few centimetres) and standard geometry of the standard insertion port(s) (and of the possible lid(s)) also have the advantages of decreasing the possibility of introducing defects in the seal during the sealing process and allowing a more reliable, possibly automated, inspection of the seal. These features ultimately lead to an oral appliance 8 that is more reliable and safer for the user.

In one embodiment, illustrated in Figure 9, the sealing between the lid 10 and the oral appliance 8 can be obtained using a sealing joint 16, as a gasket, or an O-ring, and mechanical snap-in features 17 on the lid 10.

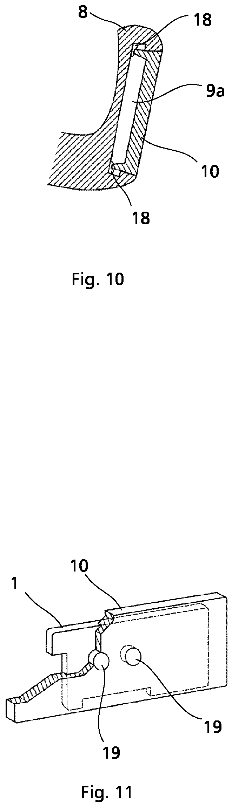

In other embodiment, illustrated in Figure 10, between the lid 10 and the oral appliance 8 can be obtained by attaching the lid 10 to the oral appliance 8 through an adhesive or gluing substance 18, for example cyanoacrylate glue, epoxy glue, hybrid glue, hotmelt, etc.

Another advantage stemming from the standard insertion port(s) and lid(s) is the possibility to use lid pre-integrated with the electronic element, for example by injection over-molding of plastic.

In one embodiment, illustrated in Figure 11, the lid 10 is made of plastic that is overmolded on a rigid part 1 of the electronic element, in such a way that electronic contacts 19 for exchanging power (for recharging the battery) and/or data are integrated in the lid 10. In other embodiments, the transfer of power and/or data is wirelessly performed.

In one embodiment, the oral appliance 8 is sealed, e.g. once the lid has been placed, and/or the liner and frame are sealed together. The sealing can be performed, e.g. by vapor smoothing, chemical smoothing, abrasive methods, heat polishing, filling materials, mechanical polishing, chemical vapour deposition processes, etc.

In one embodiment, the oral appliance 8 is sealed in correspondence of a computationally designed region. In one embodiment, the computationally designed region is at least partially extended along the interface between the liner and the frame, illustrated in Figure 6.

In one embodiment, the computationally designed region is reached by a sealant agent once the electronic module is inserted in the cavity 9.

In one embodiment, the computationally designed region is reached by a sealing agent via an opening in the appliance body. The sealing agent can be acrylic or UV curable or epoxy based or based on the same material of the oral appliance 8.

In one embodiment, the insertion port has a shape, dimensions and a spatial placement which depend on the oral anatomy of the user. For example, the insertion port is a volume completely included in the main body.

In another embodiment, an additive manufacturing device as a 3D printer creates the frame 12, the additive manufacturing is paused to insert the electronic element, and finally the additive manufacturing is completed by sealing the oral appliance. In this case, the shape and the dimensions of the insertion port are not standard.

The present invention concerns also a custom sensorized oral appliance 8 for managing sleep apnea of a user according to the invention comprises:
- a main body of the oral appliance, the main body comprising:
   - a customised cavity arranged to host a customised electronic element,
   - the customised electronic element comprising:
   - a rigid part 1,
   - a sleep apnea sensor 3, and
   - a flexible electronic interconnection arranged for electronically connect the rigid part with the sleep apnea sensor.

In this context, an electronic element is custom or customized, if the spatial placement in the electronic element of the rigid part, and/or of the sleep apnea sensor and/or the bending of the flexible electronic interconnection is(are) based on the data on the 3D oral anatomy of the user.

In one embodiment, the custom sensorized oral appliance 8 comprises a memory (not illustrated) for registering sleep apnea sensor data. In one embodiment, the memory is in the rigid part 1.

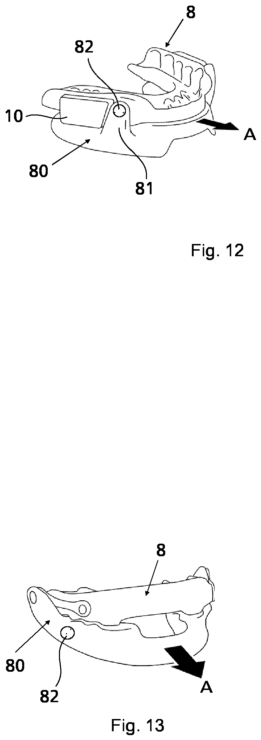

In one embodiment, the custom sensorized oral appliance 8 is a first custom sensorized oral appliance, comprising a communication module (not illustrated in the figures) arranged to send an information based on the sleep apnea sensor data to a second oral appliance 80 (visible for example in Figure 12) or to an external device, e.g. and in a non-limiting way a portable device as a smart phone.

In one embodiment, this communication module is arranged to send to the second oral appliance 80 and/or to an external device data stored in the memory of the first custom sensorized oral appliance 8.

Based on those data, the second oral appliance 80 and/or the external device are arranged to compute a sleep apnea index. Based on this index, an application or a software (e.g. run on the external device) or a person (e.g. a qualified person as a doctor) can decide if a second oral appliance 80 should be worn by the user.

In one embodiment, the second oral appliance is custom based on the 3D oral anatomy of the user and/or based on the sleep apnea sensor data from the first custom sensorized oral appliance 8.

In one embodiment, the second oral appliance 80 is arranged to treat the sleep apnea disorder, e.g. by adjusting the position of the jaw and/or tongue of the user.

The present invention concerns also a kit comprising the first custom sensorized oral appliance 8 according to the invention and at least one second oral appliance 80.

In one embodiment the second oral appliance 80 is the custom oral appliance according to the invention, with the difference that it is devoid of a sleep apnea sensor.

In one embodiment, the second oral appliance comprises a communication module (not illustrated) arranged to receive the information based on the sleep apnea sensor data from the first custom sensorized oral appliance 8.

In one embodiment, the second oral appliance comprises an actuating module (not illustrated) arranged to move the second oral appliance 8 (e.g. according to the direction of the arrow A in Figure 12) relatively to the first custom sensorized oral appliance 8, based on the received information based on the sleep apnea sensor data from the first custom sensorized oral appliance 8.

In one embodiment, the custom sensorized oral appliance uses sensors and electronic elements to detect apnea events. An algorithm, executed on a processor or microprocessor, that can be external to the custom sensorized oral appliance or belonging to the custom sensorized oral appliance, e.g. to the rigid part 1, calculates at least one apnea index (e.g. number of apnea events per hour of sleep, average duration of an apnea event, ...). Based on the analysis of the at least one apnea index, the user is instructed to use - in addition to the custom sensorized oral appliance - a second oral appliance that advances the jaw position with respect to the maxilla neutral position.

The custom sensorized oral appliance is coupled with the second appliance so that the electronic element of the custom sensorized oral appliance receives the information on the jaw advancement.

The custom sensorized oral appliance can operate together with the second appliance in order to:
- allow the user to find the most comfortable/effective advancement of the jaw position with respect to the maxilla,
- allow the user to certify that the appliance has been used with a jaw advancement, and/or
- allow the user to certify the improvement of the at least one apnea index.

In one embodiment, the biofeedback stimulus can be triggered by the custom sensorized oral appliance when used alone or in combination with the second oral appliance to reduce the residual apnea events.

In one embodiment, the second oral appliance is (numerically) designed using the geometrical information of the custom sensorized oral appliance and/or the 3D oral anatomy of the user

In one embodiment, the kit of oral appliances is obtained to offer different options of jaw advancement. The kit is (numerically) designed using the geometrical information of the custom sensorized oral appliance and/or the 3D oral anatomy of the user.

In one embodiment, the first custom sensorized oral appliance 8 and/or the second oral appliance 80 comprise(s) a coupling module 82 arranged to identify by the first custom sensorized oral appliance 8, which second appliance 80 the user is wearing from those available in the kit, and/or which level of advancement has been selected.

In one embodiment, the coupling module 82 comprises one of the following elements:
- RFID (Radio-Frequency Identification) Tags: an RFID tag embedded in one oral appliance can be read by a corresponding RFID reader in the other appliance. Each appliance can have a unique RFID tag with specific data about the device (e.g., type, model, settings). When the appliance is in proximity, the RFID reader can detect the type of the other appliance and adjust its settings accordingly.
- Magnetic Coupling with Identification: magnets integrated into each oral appliance are positioned such that they couple physically, ensuring proper alignment. Additionally, specific magnetic signatures (strength and/or polarity) can be used to identify the type of appliance. Appliances could be configured to respond differently based on the magnetic signature. In another embodiment, a magnetic material is placed in a region of the second oral appliance and the electronic element detects the resulting magnetic field and computes the relative distance and thus the jaw advancement.
- Bluetooth Low Energy (BLE) Modules: BLE modules can be integrated into each oral appliance. The appliances can communicate wirelessly to exchange data about their type, position, and settings. Once the appliances pair via BLE, one can modify its behavior (e.g., adjusting jaw position or monitoring sensor feedback) based on the presence of a specific complementary device.
- Optical Recognition Systems: optical sensors in the oral appliance can identify specific physical markers or patterns on the corresponding appliance. The markers could be a series of small, reflective elements, barcodes, or unique shape identifiers that, when recognized by an optical sensor, indicate the type of appliance. In another embodiment, the electronic element detects the relative advancement of the jaw by optically detecting the second oral appliance or a colored mark applied on it.
- Mechanical Keying or Fitment Systems: appliances can be designed with unique mechanical keying systems where only certain types of appliances physically couple or fit together based on their shape, slots, or connection points. The physical structure of the appliances ensures that only compatible devices couple together. Additionally, the keying mechanism can provide information about the type of device being used.
- Near-Field Communication (NFC) Modules: NFC is a short-range communication technology that allows two devices to exchange information when in close proximity (similar to RFID but with two-way communication). One appliance can read data from the other, such as its type, serial number, or configuration, and adjust its performance accordingly.
- Capacitive Sensing: capacitive sensors can detect the presence of an appliance based on its conductive (or proximity) properties or the specific electrical signature. These sensors can detect whether a compatible device is being used by sensing changes in capacitance when the appliances are in close contact. In another embodiment, a metallic/dielectric material is placed in a region of the second oral appliance and the electronic element detects the proximity/capacitance and computes the relative distance and thus the jaw advancement.
- Tag: A tag is placed in a region of the second oral appliance and the electronic element detects the tag and gathers the information of the jaw advancement

The above list is not limitative and other coupling modules can be used, as long as they allow for intelligent interaction between appliances, improving the overall treatment experience by ensuring the correct operation and coordination between multiple oral appliances.

In one embodiment, the kit comprises at least two (e.g. three) second custom sensorized oral appliances 80 with different lengths or advancements (titration), allowing different adjusting of the position of the jaw and/or tongue of the user.

In one embodiment, the second oral appliance 80 is arranged to indicate to the first custom sensorized oral appliance 8 the level of titration.

In one embodiment, an arm 81 is arranged to connect the first custom sensorized oral appliance 8 with the second oral appliance 80 and/or to relatively move the second oral appliance 80 with regard to the first custom sensorized oral appliance.

### Reference signs used in the figures

1 Rigid part
2a, 2b Flexible electronic interconnections
3 Sensors
4 Model of the user oral anatomy
5 Model of the rigid part
6 Model of flexible electronic interconnections
7 Model of the sensor
8 (First) oral appliance
9a Region of the cavity hosting the rigid part
9b Region of the cavity hosting flexible electronic interconnections between rigid parts
10 Lid
14 Hole
15 Electronic connector
16 Sealing joint
17 Snap-in feature of the lid
18 Adhesive
19 Electronic contacts
20 Electronic element
80 Second oral appliance
81 Arm
82 Coupling module
A Arrow

## Claims

1. A method for designing a custom sensorized oral appliance (8) for managing sleep apnea of a user, comprising the steps of:
- collect data on the 3D oral anatomy of the user,
- computationally design a main body of the oral appliance (8), the main body comprising:
- a cavity (9) arranged to host an electronic element (20), the cavity being customized on the basis of the data, and/or on the basis of a computationally designed electronic element,
- computationally design the electronic element (20), the electronic element comprising:
- a rigid part (1, 5),
- a sleep apnea sensor (3, 7) for detecting a sleep apnea event, wherein the spatial placement of the rigid part (1, 5), and/or of the sleep apnea sensor (3, 7) is(are) based on the data.

2. The method of claim 1, wherein the main body comprises an insertion port, for inserting the electronic element (20) into the main body.

3. The method of one of claims 1 or 2, wherein the insertion port is a standard insertion port, having a shape and/or dimensions which are independent on the oral anatomy of the user.

4. A method for manufacturing a custom and sensorized oral appliance (8) for managing sleep apnea of a user, comprising the steps of:
- designing the oral appliance (8) with the method for designing a custom sensorized oral appliance for managing sleep apnea of a user according to one of the claims 1 to 3,
- manufacturing the main body of the oral appliance (8) and the electronic element (20).

5. The method of claim 4, comprising the step of inserting the electronic element (20) into the main body via an insertion port.

6. The method of claim 5, comprising the step of closing the insertion port with a lid (10).

7. The method of claim 6, comprising the step of sealing the oral appliance (8) once the lid (10) has been attached.

8. Custom sensorized oral appliance (8) for managing sleep apnea of a user, comprising:
- a main body of the oral appliance, the main body comprising:
- a custom cavity (9) arranged to host a custom electronic element (20),
- the custom electronic element comprising:
- a rigid part (1), and
- a sleep apnea sensor (3).

9. The custom sensorized oral appliance (8) of claim 8, wherein the custom sensorized oral appliance (8) is a first custom sensorized oral appliance, comprising a communication module arranged to send an information based on the sleep apnea sensor data to a second oral appliance (80) or to an external device.

10. The custom sensorized oral appliance (8) of one of claims 8 to 9, comprising a feedback module.

11. The custom sensorized oral appliance (8) of one of claims 8 to 10, comprising a coupling module (82) arranged to identify a second appliance (80) and/or its advancement with regard to the first custom sensorized oral appliance.

12. Kit for managing sleep apnea comprising the first custom sensorized oral appliance (8) according to one of claims 8 or 11, and at least a second oral appliance (80).

13. The kit of claim 12, the second oral appliance (80) comprising a communication module arranged to receive the information based on the sleep apnea sensor data from the first custom sensorized oral appliance (8).

14. The kit of one of claims 12 or 13, the second oral appliance (80) comprising an actuating module arranged to move the second oral appliance (80) relatively to the first custom sensorized oral appliance (8), based on the received information based on the sleep apnea sensor data from the first custom sensorized oral appliance (8).

15. The kit of one of claims 12 to 14, the first custom sensorized oral appliance (8) and/or the second oral appliance (80) comprising a coupling module (82) arranged to identify by the first custom sensorized oral appliance (8), which second appliance (80) the user is wearing from those available in the kit.
